# EUROPEAN PATENT SPECIFICATION

(11) **EP 2 148 649 B1**
(45) Date of publication and mention of the grant of the patent: **09.02.2011**
(21) Application number: 08759859.5
(22) Date of filing: 21.05.2008
(51) Int. Cl.: A61K 8/41, A61K 8/42, A61Q 13/00

(54) **PERSONAL CARE COMPOSITIONS WITH ENHANCED FRAGRANCE DELIVERY**
KÖRPERPFLEGEZUSAMMENSETZUNGEN MIT ERHÖHTER DUFTSTOFFABGABE
COMPOSITIONS DE SOINS PERSONNELS À DIFFUSION DU PARFUM AMÉLIORÉE

(30) Priority: 30.05.2007 US 755009
(43) Date of publication of application: 03.02.2010
(73) Proprietor: Unilever PLC, A Company Registered In England And Wales under company no. 41424 of Unilever House, London EC4Y 0DY Greater London (GB); Unilever N.V., 3013 AL Rotterdam (NL)
(72) Inventor: CHANDAR, Prem, Trumbull, Connecticut 06611 (US); YANG, Lin, Trumbull, Connecticut 06611 (US)
(74) Representative: Acham, Nicholas Clive
(86) International application number: PCT/EP2008/056257
(87) International publication number: WO 2008/145582

(56) References cited:
- WO-A-2004/024114
- WO-A-2006/018198
- WO-A-2006/045583
- WO-A-2006/045584
- DE-C1- 19 517 048
- US-A- 4 978 526

## Description

### BACKGROUND OF THE INVENTION

The invention concerns personal care compositions which upon application to a human body surface quickly release fragrance components thereby improving aesthetics of these compositions.

Perhaps the most significant aesthetic of a personal care product for a consumer is fragrance. It is also important to rapidly deliver the scent.

Many techniques have been reported to manipulate timing and impact of fragrance. Delayed generation has been achieved through encapsulation of scent ingredients. For instance, U.S. Patent 5,135,747 (Faryniarz et al.) reports an unscented malodor counteractant deo perfume mixture encapsulated within a semi-perrneable wall material and a quicker releasable non-encapsulated fragrance perfume mixture in a cosmetically acceptable vehicle. Slow release has also been achieved through pro-accords. These chemicals slowly break down releasing an odoriferous component as a degradation fragment. Menthol is the most frequent commercially delivered degradation constituent of pro-accords contained in personal care compositions. Illustrative of this technology is U.S. Patent 6,100,233 (Sivik et al.) employing a β-ketoester pro-accord which transforms to chemically release fragranced alcohols such as linalool, dihydromyrcenol and other alcohols.

Steady release technologies have also been reported. Most prominent are a series of disclosures on enduring perfumes. See U.S. Patent 5,833,999; U.S. Patent 5,849,310 and U.S. Patent 6,086,903 all to Trinh et al. describing personal treatment compositions delivering an enduring perfume that provides a lasting olifactory sensation.

Although technologies are known for delayed release and prolonged perfume generation, none has solved the problem of rapidly releasing a fragrance onto human skin or hair.

### SUMMARY OF THE INVENTION

A personal care composition is provided which includes:
(i) from 0.0001 to 5% of a fragrance to impart a pleasant scent onto a human body to which the composition is applied;
(ii) from 0.01 to 20% of a substituted urea of general structure (I) 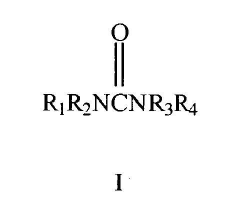 wherein R₁, R₂ and R₃ are selected from the group consisting of hydrogen, C₁-C₆ alkyl, (R₅)ₙOH, and mixtures thereof; R₅ is methylene, ethylene, propylene or combinations thereof, and n ranges from 1 to 6; and R₄ is (R₅)ₙOH; and
(iii) from 0.01 % to 20% by weight of a dihydroxypropyl trialkyl quaternary ammonium salt of structure AB, wherein A is a cationic charged component of the salt AB, B is an anionic charged component of the salt AB, and A has a single quaternized nitrogen atom, at least two hydroxy groups and a molecular weight no higher than 250.

### DETAILED DESCRIPTION OF THE INVENTION

Now it has been found that a burst of fragrance from a personal care composition can be quickly released when the composition is deposited upon a human body part. More particularly, a combination of a substituted urea and a dihydroxypropyl quaternary ammonium salt function as scent boosting agents. These agents allow rapid volatilization of fragrance components during application of the personal care composition to skin or hair.

By the term personal care composition is meant any product applied to a human body for improving appearance, cleansing, odor control or general aesthetics. Non-limiting examples of personal care compositions include leave-on skin lotions and creams, shampoos, conditioners, shower gels, toilet bars, antiperspirants, deodorants, shave creams, depilatories, lipsticks, foundations, mascara, sunless tanners and sunscreen lotions.

An important element of the present invention is a first scent boosting agent. This is a substituted urea having general structure (I) 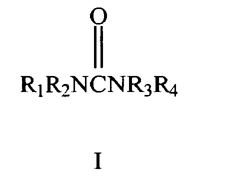 wherein R₁, R₂ and R₃ are selected from the group consisting of hydrogen, C₁-C₆ alkyl, (R₅)ₙOH, and mixtures thereof; R₅ is methylene, ethylene, propylene or combinations thereof, and n ranges from 1 to 6; and R₄ is (R₅)ₙOH.

Illustrative species of the substituted urea are hydroxymethyl urea, hydroxyethyl urea, hydroxypropyl urea, bis(hydroxymethyl) urea, bis(hydroxyethyl) urea, bis(hydroxypropyl) urea, N,N'-di-hydroxymethyl urea, N,N'-di-hydroxyethyl urea, N,N'-di-hydroxypropyl urea, N,N,N'-tri-hydroxyethyl urea, tetra(hydroxymethyl) urea, tetra(hydroxyethyl) urea, tetra(hydroxypropyl) urea, N-methyl-N'-hydroxyethyl urea, N-ethyl-N'-hydroxyethyl urea, N-hydroxypropyl-N'-hydroxyethyl urea and N,N'-dimethyl-N-hydroxyethyl urea. Where the term hydroxypropyl appears, the meaning is generic for either 3-hydroxy-n-propyl, 2-hydroxy-n-propyl, 3-hydroxy-i-propyl or 2-hydroxy i-propyl radicals. Most preferred is hydroxyethyl urea. The latter is available as a 50% aqueous liquid from the National Starch & Chemical Division of ICI under the trademark Hydrovance.

Amounts of the substituted urea were may range from 0.01 to 20%, preferably from 0.5 to 15%, more preferably from 1 to 10%, and optimally from 5 to 8% by weight of the personal care composition.

A second scent boosting agent of the present invention is a dihydroxypropyl trialkyl quaternary ammonium salt of structure AB, wherein A is a cationic charged component of the salt AB, and B is an anionic charged component of the salt AB, A has one quatemized nitrogen atom, at least two hydroxyl groups and a molecular weight no higher than 250 but preferably no higher than 200, and optimally no higher than 170.

Anionic charged component B may be organic or inorganic with proviso that the material is cosmetically acceptable. Typical inorganic anions are halides, sulfates, phosphates, nitrates and borates. Most preferred are the halides, especially chloride. Organic anionic counter ions indude methosulfate, toluoyl sulfate, acetate, dtrate, tartrate, lactate, gluconate and benzenesulfonate. The number and charge of negatively charged component B will be sufficient to neutralize the positive charge of component A

A preferred embodiment of the quaternary ammonium saft is the dihydroxypropyl tri(C₁-C₃ alkyl) ammonium salts.

These salts may be obtained in a variety of synthetic procedures, most particularly by hydrolysis of chlorohydroxypropyltri(C₁-C₃ alkyl) ammonium salts. Ordinarily the C₁-C₃ alkyl constituent on the quatemized ammonium group will be methyl, ethyl, n-propyl, isopropyl and mixtures thereof. Particularly preferred is a trimethyl ammonium group known through INCl nomenclature as a "trimonium" group. A most preferred species is 1,2-dihydroxypropyltrimonium chloride, wherein the C₁-C₃ alkyl is a methyl group.

Amounts of the quaternary ammonium salts may range from 0.01 to 20%, preferably from 0.5 to 15%, more preferably from 1 to 10%, optimally from 5 to 8% by weight of the personal care composition.

The term "fragrance" is defined as a mixture of odoriferous components, optionally mixed with a suitable solvent diluent or carrier, which is employed to impart a desired odor.

Fragrance components and mixtures thereof may be obtained from natural products such as essential oils, absolutes, resinoids, resins and concretes, as well as synthetic products such as hydrocarbons, alcohols, aldehydes, ketones, ethers, carboxylic adds, esters, acetals, ketals, nitriles and the like, including saturated and unsaturated compounds, aliphatic, carbocyclic and heterocyclic compounds.

Suitable characteristics of fragrances can indude at least one of the following, in any combination: (1) liquid or semi-liquid after mixing with the other components; (2) pleasant and/or dean odor when mixed with other components, e.g. one or more of lavender, violet, rose, jasmin, pine, woody, floral, fruity, lemon, lime, apple, peach, raspberry, strawberry, banana, plum, apricot, vanilla, pear, eucalyptus, aromatic, aldehydic, tutti frutti, oriental, sweet, amber, Paola, Muguet, Citronella (lime), and the like; (3) specific gravity (20/20) in the range of 0.600-1.300, preferably 0.800-1.100, each preferably varying 0.001-0.05, more preferably 0.008-0.020; (4) refractive index (20°C) of 1.300-1.800, preferably 1.400-1.600, each preferably varying 0.001-0.05, more preferably 0.008-0.020; (5) saponification value of 5-300, preferably 10-250; and (6) having a flash point of 20-200 Pensky-Martens Closed Cup (P.M.C.C.) and 10-100 Tag-Closed Cup (T.C.C.).

Typical fragrance components which may be employed for the present invention can be selected from one or more of: 2-methoxy naphthalene; allyl cyclohexane propionate; alpha-citronellal; alpha-ionone; alpha-santalol; alpha-tierpineol; ambrettolide; amyl benzoate; amyl dnnamate; amyl cinnamic aldehyde; aurantiol; benzaldehyde; benzophenone; benzyl acetate; benzyl salicylate; beta-caryophytiene; beta-methyl naphthyl ketone; cadinene; cavacrol; cedrol; cedryl acetate; cedryl formate; cinnamyl dnnamate; cis-jasmone; coumarin; cyclamen aldehyde; cyclohexyl salicylate; d-limonene; delta-nonalactone; delta-undecalactone; dihydro isojasmonate; dihydro mycenol; dimethyl acetal; diphenyl methane; diphenyl oxide; dodecalactone; ethyl methyl phenyl glycidate; ethyl undecylenate; ethylene brassylate; eugenol; exaltolide; galaxolide; gamma-n-methyl ionone; gamma-undecalactone; geranial; geranyl acetate; geranyl anthranilate; geranyl phenyl acetate; hexadecanolide; hexenyl salicylate; hexyl cinnamic aldehyde; hexyl salicylate; hydroxycitronellal; indole; iso E super; iso-amyl salicylate; iso-bomyl acetate; iso-butyl quinoline; iso-eugenol; laevo-carvone; lilial (p-t-budnal); linalool; linalyl acetate; linalyl benzoate; methyl dnnamate; methyl dihydrojasmonate; methyl-N-methyl anthranilate; musk indanone; musk ketone; musk tibetine; myristicin; nerol; oxahexadecanolide-10; oxahexadecanolide-11; para-cymene; para-tert-butyl cyclohexyl acetate;; patchouli alcohol; phantolide; phenyl ethyl alcohol; phenyl ethyl benzoate; phenyl heptanol; phenylhexanol; phexylethylphenylacetate; thibetolide; vanillin; vertenex; vetiveryl acetate; yara-yara; and ylangene.

Suitable solvents, diluents or carriers for perfumes as mentioned above are, for example, ethanol, isopropanol, diethylene glycol monoethyl ether, dipropyl glycol, triethyl citrate and the like.

Particularly preferred fragrance components of the present invention are cyclic and acyclic terpenes and terpenoids. These materials are based upon isoprene repeating units. Examples include alpha and beta pinene, myrcene, geranyl alcohol and acetate, camphene, dl-limonene, alpha and beta phellandrene, tricyclene, terpinolene, allocimmane, geraniol, nerol, linanool, dihydrolinanool, citral, ionone, methyl ionone, citronellol, citronellal, alpha terpineol, beta terpineol, alpha fenchol, borneol, isoborneol, camphor, terpinen-1-ol, terpin-4-ol, dihydroterpineol, methyl chavicol, anethole, 1,4- and 1,8-cineole, geranyl nitrile, isobomyl acetate, linalyl acetate, caryophyllene, alpha cedrene, guaiol, patchouli alcohol, alpha and beta santalol and mixtures thereof.

Amounts of the fragrance may range from 0.0001 to 5%, usually from 0.001 to 1.5%, more usually from 0.5 to 0.8% by weight of the personal care composition.

Compositions of this invention may also indude a cosmetically acceptable carrier. Amounts of the carrier may range from 1 to 99.9%, preferably from 70 to 95%, optimally from 80 to 90% by weight of the composition. Among the useful carriers are water, emollients, fatty acids, fatty alcohols, humectants, thickeners and combinations thereof. The carrier may be aqueous, anhydrous or an emulsion. Preferably the compositions are aqueous, especially water and oil emulsions of the W/O or O/W or triplex W/O/W variety. Water when present may be in amounts ranging from 5 to 95%, preferably from 20 to 70%, optimally from 35 to 60% by weight of the personal care composition.

Emollient materials may serve as cosmetically acceptable carriers. These may be in the form of silicone oils, synthetic esters and hydrocarbons. Amounts of the emollients may range anywhere from 0.1 to 95%, preferably between 1 and 50% by weight of the personal care composition.

Silicone oils may be divided into the volatile and non volatile variety. The term "volatile" as used herein refers to those materials which have a measurable vapor pressure at ambient temperature. Volatile silicone oils are preferably chosen from cyclic (cydomethicone) or linear polydimethylsiloxanes containing from 3 to 9, preferably from 4 to 5, silicon atoms.

Non-volatile silicone oils useful as an emollient material indude polyalkyl siloxanes, polyalkylaryl siloxanes and polyether siloxane copolymers. The essentially non volatile polyalkyl siloxanes useful herein include, for example, polydimethyl siloxanes with viscosities of from 5 x 10-6 to 0.1 m²/s at 25°C. Among the preferred non volatile emollients useful in the present compositions are the polydimethyl siloxanes having viscosities from 1 x 10⁻⁵ to 4 x 10⁻⁴ m²/s at 25°C. Another class of non-volatile silicone oils are emulsifying and non-emulsifying silicone elastomers. Representative of this category is Dimethicone/Vinyl Dimethicone Crosspolymer available as Dow Coming 9040, General Electric SFE 839, and Shin-Etsu KSG-18. Silicone waxes such as Silwax WS-L (Dimethicone Copolyol Laurate) may also be useful.

Among the ester emollients are:
1) Alkyl esters of saturated fatty acids having 10 to 24 carbon atoms. Examples thereof include behenyl neopentanoate, isononyl isonanonoate, isopropyl myristate and octyl stearate.
2) Ether-esters such as fatty acid esters of ethoxylated saturated fatty alcohols.
3) Polyhydric alcohol esters. Ethylene glycol mono- and di-fatty add esters, diethylene glycol mono- and di-fatty acid esters, polyethylene glycol (200-6000) mono- and di-fatty add esters, propylene glycol mono- and di-fatty acid esters, polypropylene glycol 2000 monostearate, ethoxylated propylene glycol monostearate, glyceryl mono- and di-fatty add esters, polyglycerol poly-fatty esters, ethoxylated glyceryl mono-stearate, 1,3-butylene glycol monostearate, 1,3-butylene glycol distearate, polyoxyethylene polyol fatty add ester, sorbitan fatty add esters, and polyoxyethylene sorbitan fatty add esters are satisfactory polyhydric alcohol esters. Particularly useful are pentaerythritol, trimethylolpropane and neopentyl glycol esters of C₁-C₃₀) alcohols.
4) Wax esters such as beeswax, spermaceti wax and tribehenin wax.
5) Sugar ester of fatty adds such as sucrose polybehenate and sucrose polycottonseedate.

Hydrocarbons which are suitable cosmetically acceptable carriers indude petrolatum, mineral oil, C₁₁-C₁₃ isoparaffins, and especially isohexadecane, available commercially as Permethyl 101A from Presperse Inc.

Fatty acids having from 10 to 30 carbon atoms may also be suitable as cosmetically acceptable carriers. Illustrative of this category are pelargonic, lauric, myristic, palmitic, stearic, isostearic, oleic, hydroxystearic and behenic acids.

Fatty alcohols having from 10 to 30 carbon atoms are another useful category of cosmetically acceptable carrier. Illustrative of this category are stearyl alcohol, lauryl alcohol, myristyl alcohol and cetyl alcohol.

Humectants of the polyhydric alcohol-type can be employed as cosmetically acceptable carriers. Typical polyhydric alcohols include glycerol, polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, hexylene glycol, 1,3-butylene glycol, isoprene glycol, 1,2,6-hexanetriol, ethoxylated glycerol, propoxylated glycerol and mixtures thereof. The amount of humectant may range anywhere from 0.5 to 50%, preferably between 1 and 15% by weight of the personal care composition.

Thickeners can be utilized as part of the cosmetically acceptable carrier of compositions according to the present invention. Typical thickeners indude crosslinked acrylates (e.g. Carbopol 982®), hydrophobically-modified acrylates (e.g. Carbopol 1382®), cellulosic derivatives and natural gums. Among useful cellulosic derivatives are sodium carboxymethylcellulose, hydroxypropyl methocellulose, hydroxypropyl cellulose, hydroxyethyl cellulose, ethyl cellulose and hydroxymethyl cellulose. Natural gums suitable for the present invention include guar, xanthan, sclerotium, carrageenan, pectin and combinations of these gums. Inorganics may also be utilized as thickeners, particularly days such as bentonites and hectorites, fumed silicas, and silicates such as magnesium aluminum silicate (Veegum®). Amounts of the thickener may range from 0.0001 to 10%, usually from 0.001 to 1%, optimally from 0.01 to 0.5% by weight of the personal care composition.

Personal care compositions of the present invention may be in any form. These forms may indude lotions, creams, roll-on formulations, sticks, mousses, aerosol and non-aerosol sprays and fabric (e.g. non-woven textile)-applied formulations.

Surfactants may also be present in compositions of the present invention. Total concentration of the surfactant when present may range from 0.1 to 40%, preferably from 1 to 20%, optimally from 1 to 5% by weight of the personal care composition. The surfactant may be selected from the group consisting of anionic, nonionic, cationic and amphoteric actives. Particularly preferred nonionic surfactants are those with a C₁₀-C₂₀ fatty alcohol or acid hydrophobe condensed with from 2 to 100 moles of ethylene oxide or propylene oxide per mole of hydrophobe, C₂C₁₀ alkyl phenols condensed with from 2 to 20 moles of alkylene oxide, mono- and di-fatty acid esters of ethylene glycol, fatty add monoglyceride; sorbitan, mono- and di- C₈-C₂₀ fatty acids, and polyoxyethylene sorbitan as well as combinations thereof. Alkyl polyglycosides and saccharide fatty amides (e.g. methyl gluconamides) are also suitable nonionic surfactants.

Preferred anionic surfactants indude soap, alkyl ether sulfates and sulfonates, alkyl sulfates and sulfonates, alkylbenzene sulfonates, alkyl and dialkyl sulfosuccinates, C₈-C₂₀ acyl isethionate, C₈C₂₀ alkyl ether phosphates, C₈-C₂₀ sarcosinates and combinations thereof.

Sunscreen actives may also be included in compositions of the present invention. Particularly preferred are such materials as ethylhexyl p-methoxydnnamate (available as Parsol MCX®), Avobenzene (available as Parsol 1789®), octylsalicylate (available as Dermablock OS®), tetraphthalylidene dicamphor sulfonic acid (available as Mexoryl SX®), benzophenone-4 and benzophenone-3 (Oxybenzone). Inorganic sunscreen actives may be employed such as microfine titanium dioxide, zinc oxide, polyethylene and various other polymers. By the term "microfine" is meant particles of average size ranging from 10 to 200 nm, preferably from 20 to 100 nm. Amounts of the sunscreen agents when present may generally range from 0.1 to 30%, preferably from 2 to 20%, optimally from 4 to 10% by weight of the personal care composition.

Preservatives can desirably be incorporated into the cosmetic compositions of this invention to protect against the growth of potentially harmful microorganisms. Suitable traditional preservatives for compositions of this invention are alkyl esters of para-hydroxybenzoic add. Other preservatives which have more recently come into use indude hydantoin derivatives, propionate salts, and a variety of quaternary ammonium compounds. Cosmetic chemists are familiar with appropriate preservatives and routinely choose them to satisfy the preservative challenge test and to provide product stability. Particularly preferred preservatives are phenoxyethanol, methyl paraben, propyl paraben, imidazolidinyl urea, sodium dehydroacetate and benzyl alcohol. The preservatives should be selected having regard for the use of the composition and possible incompatibilities between the preservatives and other ingredients in the emulsion. Preservatives are preferably employed in amounts ranging from 0.01% to 2% by weight of the personal care composition.

Compositions of the present invention may include vitamins. Illustrative vitamins are vitamin A (retinol), vitamin B₂, vitamin B₃ (niacinamide), vitamin B₆, vitamin C, vitamin E and biotin. Derivatives of the vitamins may also be employed. For instance, vitamin C derivatives indude ascorbyl tetraisopalmitate, magnesium ascorbyl phosphate and ascorbyl glycoside. Derivatives of vitamin E indude tocopheryl acetate, tocopheryl palmitate and tocopheryl linoleate. DL-panthenol and derivatives may also be employed. A particularly suitable vitamin B₆ derivative is pyridoxine palmitate. Flavanoids may also be useful, particularly glucosyl hesperidin, rutin, and soy isoflavones (induding genistein, daidzein, equol, and their glucosyl derivatives) and mixtures thereof. Total amount of vitamins or flavonoids when present may range from 0.0001 to 10%, preferably from 0.01 % to 1%, optimally from 0.1 to 0.5% by weight of the composition personal care

Another type of useful substance can be that of an enzyme such as oxidases, proteases, lipases and combinations. Particularly preferred is superoxide dismutase, commercially available as Biocell SOD from the Brooks Company, USA.

Skin lightening compounds may be included in the compositions of the invention. Illustrative substances are placental extract, lactic acid, niadnamide, arbutin, kojic add, ferulic add, resorcinol and derivatives including 4-substituted resorcinols and combinations thereof. Amounts of these agents may range from 0.1 to 10%, preferably from 0.5 to 2% by weight of the personal care composition.

Desquamation promoters may be present Illustrative are the alpha-hydroxycarboxylic adds and beta-hydroxycarboxylic adds. The term "acid" is meant to include not only the free add but also salts and C₁-C₃₀ alkyl or aryl esters thereof and lactones generated from removal of water to form cyclic or linear lactone structures. Representative adds are glycolic, lactic and malic adds. Salicylic add is representative of the beta-hydroxycarboxylic acids. Amounts of these materials when present may range from 0.01 to 15% by weight of the personal care composition.

A variety of herbal extracts may optionally be included in compositions of this invention. Illustrative are pomegranate, white birch (Betula Alba), green tea, chamomile, licorice and extract combinations thereof. The extracts may either be water soluble or water-insoluble carried in a solvent which respectively is hydrophilic or hydrophobic. Water and ethanol are the preferred extract solvents.

Also included may be such materials as lipoic acid, kinetin, retinoxytrimethylsilane (available from Clariant Corp. under the Silcare 1M-75 trademark), dehydroepiandrosterone (DHEA) and combinations thereof. Ceramides (induding Ceramide 1, Ceramide 3, Ceramide 3B, Ceramide 6 and Ceramide 7) as well as pseudoceramides may also be utilized for many compositions of the present invention but may also be excluded. Amounts of these materials may range from 0.000001 to 10%, preferably from 0.0001 to 1 % by weight of the personal care composition.

Colorants, pacifiers and abrasives may also be included in compositions of the present invention. Each of these substances may range from 0.05 to 5%, preferably between 0.1 and 3% by weight of the personal care composition.

The compositions of the present invention can also be, optionally, incorporated into a water insoluble substrate for application to the skin such as in the form of a treated wipe.

The following examples will more fully illustrate the embodiments of this invention. All parts, percentages and proportions referred to herein and in the appended claims are by weight unless otherwise illustrated.

### EXAMPLE 1

A representative personal care composition of the present invention in the form of a cosmetic lotion is outlined under table I.

**TABLE I**

| INGREDIENT | WEIGHT % |
|---|---|
| PHASE A | |
| Water | Balance |
| Disodium EDTA | 0.05 |
| Methyl paraben | 0.15 |
| Magnesium aluminum silicate | 0.60 |
| Triethanolamine | 1.20 |
| Hydroxyethyl urea | 1.00 |
| Dihydroxypropytrimonium chloride | 1.00 |

| PHASE B | |
|---|---|
| Xanthan gum | 0.20 |
| Natrosol® 250HHR (ethyl cellulose) | 0.50 |
| Butylene glycol | 3.00 |
| Glycerin | 2.00 |

| PHASE C | |
|---|---|
| Sodium stearoyl lactylate | 0.10 |
| Glycerol monostearate | 1.50 |
| Stearyl alcohol | 1.50 |
| Isostearyl palmitate | 3.00 |
| Silicone fluid | 1.00 |
| Cholesterol | 0.25 |
| Sorbitan stearate | 1.00 |
| Butylated hydroxy toluene | 0.05 |
| Vitamin E acetate | 0.01 |
| PEG-100 stearate | 2.00 |
| Stearic acid | 3.00 |
| Propyl paraben | 0.10 |
| Parsol MCX® | 2.00 |
| Caprylic/Capric triglyceride | 0.50 |
| Hydroxycaprylic acid | 0.01 |
| C12-15 Alkyl octanoate | 3.00 |

| PHASE D | |
|---|---|
| Vitamin A palmitate | 0.10 |
| Bisabolol | 0.01 |
| Vitamin A acetate | 0.01 |
| Fragrance | 1.00 |
| Retinol 50C | 0.02 |
| Conjugated linoleic acid | 0.50 |

### EXAMPLE 2

A water-in-oil topical liquid make-up foundation according to invention is described in table II below.

**TABLE II**

| INGREDIENT | WEIGHT % |
|---|---|
| PHASE A | |
| Cyclomethicone | 9.25 |
| Oleyl oleate | 2.00 |
| Dimethicone copolyol | 20.00 |

| PHASE B | |
|---|---|
| Talc | 3.38 |
| Pigment (iron oxides) | 10.51 |
| Spheron L-1500 (silica) | 0.50 |

| PHASE C | |
|---|---|
| Synthetic wax Durachem 0602 | 0.10 |
| Arachidyl behenate | 0.30 |

| PHASE D | |
|---|---|
| Cyclomethicone | 1.00 |
| Trihydroxystearin | 0.30 |

| PHASE E | |
|---|---|
| Laureth-7 | 0.50 |
| Propyl paraben | 0.25 |

| PHASE F | |
|---|---|
| Fragrance | 0.5 |

| PHASE G | |
|---|---|
| Water | balance |
| Hydroxymethyl urea | 3.00 |
| Dihydroxypropyltrimonium chloride | 1.00 |
| Methyl paraben | 0.12 |
| Propylene glycol | 8.00 |
| Niacinamide | 4.00 |
| Glycerin | 3.00 |
| Sodium chloride | 2.00 |
| Sodium dehydroacetate | 0.30 |

### EXAMPLE 3

A disposable, single use personal care towelette product is described according to the present invention. A 70/30 polyester/rayon non-woven towelette is prepared with a weight of 1.8 grams and dimensions of 15 cm by 20 cm. Onto this towelette is impregnated a composition with a terpenoid type fragrance, a substituted urea and dihydroxypropyltrimonium chloride as outlined in table III below.

**TABLE III**

| INGREDIENT | WEIGHT % |
|---|---|
| Hydroxyethyl urea (50% in water) | 7.50 |
| Dihydroxypropyltrimonium chloride | 4.00 |
| Glycerin | 2.00 |
| Hexylene glycol | 2.00 |
| Disodium capryl amphodiacetate | 1.00 |
| Gluconolactone | 0.90 |
| Silicone microemulsion | 0.85 |
| Witch hazel | 0.50 |
| PEG-40 Hydrogenated castor oil | 0.50 |
| Fragrance (terpenoid mixture) | 0.20 |
| Vitamin E acetate | 0.001 |
| Water | Balance |

### EXAMPLE 4

A toilet bar illustrative of the present invention is outlined under table IV.

**TABLE IV**

| INGREDIENT | WEIGHT % |
|---|---|
| Sodium soap (85/15 tallow/coconut) | 77.77 |
| Dihydroxypropyltrimonium chloride | 3.50 |
| Hydroxyethyl urea | 1.00 |
| Dimethicone | 2.00 |
| Sodium chloride | 0.77 |
| Titanium dioxide | 0.40 |
| Fragrance | 1.50 |
| Disodium EDTA | 0.02 |
| Sodium etidronate | 0.02 |
| Fluorescer | 0.024 |
| Water | Balance |

### EXAMPLE 5

A shampoo composition useful in the context of the present invention is described in table V below.

**TABLE V**

| Ingredient | Weight % |
|---|---|
| Ammonium laureth sulfate | 12.00 |
| Ammonium lauryl sulfate | 2.00 |
| Cocoamidopropyl betaine | 2.00 |
| Sodium lauroamphoacetate | 2.00 |
| Glycerin | 12.00 |
| Dihydroxypropyltrimonium chloride | 5.50 |
| Hydroxyethyl urea | 1.50 |
| Ethylene glycol distearate | 1.50 |
| Cocomonoethanolamide | 0.80 |
| Cetyl alcohol | 0.60 |
| Polyquatemium-10 | 0.50 |
| Dimethicone | 1.00 |
| Zinc pyridinethione | 1.00 |
| Sodium citrate | 0.40 |
| Citric acid | 0.39 |
| Sodium xylene sulfonate | 1.00 |
| Fragrance | 0.40 |
| Sodium benzoate | 0.25 |
| Kathon CG® | 0.0008 |
| Benzyl alcohol | 0.0225 |
| Water | Balance |

### EXAMPLE 6

This example illustrates an antiperspirant/deodorant formula according to the present invention.

**TABLE VI**

| Ingredient | Weight % |
|---|---|
| Cyclopentasiloxane | 36 |
| Dimethicone | 20 |
| Aluminum zirconium trichlorohydrex glycinate | 15 |
| Dihydroxypropyltrimonium chloride | 5.0 |
| Hydroxyethyl urea | 3.0 |
| C₁₈-C₃₆ Acid triglyceride | 5.0 |
| Microcrystalline wax | 3.0 |
| Glycerin | 8.0 |
| Silica | 2.5 |
| Dimethicone crosspolymer | 1.0 |
| Fragrance | 0.5 |
| Disodium EDTA | 0.4 |
| Butylated hydroxytoluene | 0.3 |
| Citric Acid | 0.3 |

### EXAMPLE 7

This example illustrates a series of skin lotions formulated for purposes of the present invention.

**TABLE VII**

| | Sample Number (Weight %) | | | | |
|---|---|---|---|---|---|
| Ingredients | A | B | C | D | E |
| PEG-4 | - | qs | - | - | - |
| PEG-8 | qs | - | qs | qs | - |
| Hydroxyethyl urea | 2 | 2 | 2 | 2 | 2 |
| Dihydroxypropyl trimonium chloride | 5 | 5 | 5 | 5 | 5 |
| Propylene glycol | - | - | 5 | - | qs |
| Dipropylene glycol | - | - | 10 | - | - |
| Pentylene glycol | - | - | 5 | - | - |
| Glycerin | - | - | - | 10 | - |
| Water | - | - | - | 5 | - |
| Sodium bicarbonate | 34 | 15 | - | 20 | 25 |
| Magnesium sulfate | - | - | 15 | - | - |
| Dryflo Plus¹ | - | - | - | 2 | - |
| Microthene FN510-00² | - | - | 2 | - | - |
| Titanium dioxide | - | - | - | 0.5 | - |
| Cab-O-Sil (Fumed Silica) | - | 4 | - | - | - |
| Niadnamide | 0.1 | - | - | - | 0.1 |
| D-Panthenol | - | 0.5 | - | - | - |
| Vitamin C | 0.001 | - | - | - | - |
| Vitamin E acetate | 0.01 | - | - | - | - |
| Polysorbate 20 | 4 | - | 6 | - | - |
| Laureth-4 | - | - | - | 0.5 | - |
| Methylparaben | - | - | - | 0.05 | - |
| FD & C Dyes | 0.0011 | - | - | - | - |
| Fragrance | 0.15 | 0.8 | 12 | 3 | 5 |

| | | | | | |
|---|---|---|---|---|---|
| Aluminum starch octenylsuccinate powder supplied by National Starch, Bridgewater, NJ, USA 2 Polyethylene powder supplied Equistar Chemicals, Houston, TX, USA | | | | | |

### EXAMPLE 8

A series of experiments were conducted to evaluate release and prolonged scent generation of typical components of a perfume mixture. Samples were prepared at room temperature (23°C) by mixing 10% by weight of the sample of scent boosting agent (substituted urea and/or dihydroxypropyl quaternary ammonium salt) in water along with 0.05% by weight of the sample Deep Moisture perfume oil. This oil is a mixture of components including but not limited to limonene, dihydromyrcenol, gamma terpinene, benzyl acetate, linalool, pinene, isomethyl ionone and others.

Samples were analyzed by gas chromatography (GC) analysis of headspace gases. In this procedure, the equipment utilized was a solid phase microextraction (SPME) system employing an Agilent gas chromatography (GC) 6890 / mass spectrometry (MS) 5973 / flame ionization detector (FID). This equipment measured relative perfume compound abundance in the headspace over the fragrance/boosting agent/water mixture, as well as over the fragrance/water mixture. One gram of fragrance/boosting agent/water mixture was prepared in 20 ml GC headspace sampling vials sealed with caps having septums (from Gerstel, Inc.) and held at room temperature (23°C). The GC column was a HP-5MS column from Agilent (inner diameter 0.25 mm, length 30 m, stationary phase thickness 0.25um). The GC conditions were as follows: Injector in splitless mode with helium gas as carrier gas. Injection port was heated to 250°C, purge flow to split vent 50ml/min at zero minutes. Column was in constant flow mode with 1.3 ml/min flow rate. Oven temperature ramp: hold at 75°C for 2 minutes, then increase oven temperature at a rate of 6°C/min to 100°C, 1.5°C/min to 150°C, 3°C/,min to 190°C, 30°C/min to 300°C and hold for 2 minutes. MS conditions were: solvent delay for 0.5 minutes, scan starting from low mass 35 to high mass 300. Autosampler's conditions were: No incubation (all experiments done in room temperature). SPME fibre was inserted into the sample headspace for a 5 minute extraction and then injected to the injector for a 15 minute desorption.

Results of the experiments are reported as relative fragrance component headspace abundance in table VIII below.

Vaporization of all fragrance components for the combination of 5% GQ/5% HEU were better, and in some instances exceptionally better than the water base. With just a few exceptions, the combination of GQ/HEU provided a better result than GQ or HEU separately. From these results, it is evident that a combination of dihydroxypropyltrimonium chloride and hydroxyethyl urea (GQ/HEU) operated to boost scent into the headspace above the aqueous base formula.

**TABLE VIII**

| Fragrance Component | Water | 10% GQ | 10% HEU | 5% GQ/5% HEU |
|---|---|---|---|---|
| 1-Butanol, 3-methyl acetate | 1.00 | 1.09 | 0.84 | 1.05 |
| 2-Buten-1-ol, 3-methyl acetate | 1.00 | 1.04 | 0.93 | 1.22 |
| Beta pinene | 1.00 | 1.72 | 1.94 | 2.76 |
| Hexyl acetate | 1.00 | 1.41 | 1.34 | 1.48 |
| Limonene | 1.00 | 2.72 | 2.90 | 2.57 |
| 2,6 dimethyl hept-5-en-1-al | 1.00 | 0.38 | 1.06 | 1.10 |
| Gamma terpinene | 1.00 | 2.15 | 3.33 | 3.18 |
| Dihydromyrcenol | 1.00 | 1.00 | 0.97 | 1.17 |
| 2,4 Dimethyl-3-cydohexene-1-carbaldehyde | 1.00 | 1.06 | 0.90 | 1.11 |
| Linalool | 1.00 | 0.97 | 1.01 | 1.20 |
| Benzyl acetate | 1.00 | 0.86 | 0.98 | 1.15 |
| Allyl heptoate | 1.00 | 1.2 | 1.74 | 1.96 |
| 2-Tertiobutylcyclohexyl acetate-2 | 1.00 | 1.16 | 1.48 | 1.57 |
| Alpha isomethyl ionone | 1.00 | 0.86 | 1.26 | 1.36 |
| Lily aldehyde | 1.00 | 0.97 | 1.32 | 1.54 |

| | | | | |
|---|---|---|---|---|
| GQ = dihydroxypropyltrimonium chloride HEU = hydroxyethyl urea | | | | |

## Claims

1. A personal care composition comprising:
(i) from 0.0001 to 5% of a fragrance to impart a pleasant scent onto a human body to which the composition is applied;
(ii) from 0.01 to 20% of a substituted urea of general structure (I) 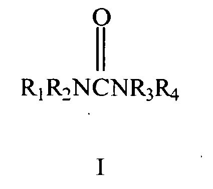 wherein R₁, R₂ and R₃ are selected from the group consisting of hydrogen, C₁-C₆ alkyl, (R₅)ₙOH, and mixtures thereof; R₅ is methylene, ethylene, propylene or combinations thereof, and n ranges from 1 to 6; and R₄ is (R₅)ₙOH; and
(iii) from 0.01% to 20% by weight of a dihydroxypropyl trialkyl quaternary ammonium salt of structure AB, wherein A is a cationic charged component of the salt AB, B is an anionic charged component of the salt AB, and A has a single quaternized nitrogen atom, at least two hydroxy groups and a molecular weight no higher than 250.

2. A composition according to claim 1 wherein the quaternary ammonium salt is a dihydroxypropyltri(C₁-C₃ alkyl) ammonium salt.

3. A composition according to claim 2 wherein the salt is dihydroxypropyltrimonium chloride.

4. A composition according to any one of the preceding claims which is selected from the group consisting of leave-on skin lotions and creams, shampoos, hair conditioners, shower gels, toilette bars, antiperspirants, deodorants, dental products, shave creams, depilatories, lipsticks, foundations, mascara, sunless tanner and sunscreen lotions.

5. A composition according to any one of the preceding claims wherein the substituted urea is present in an amount from 0.05 to 15% by weight of the personal care composition.

6. A composition according to any one of the preceding claims wherein the substituted urea is hydroxyethyl urea.

7. A composition according to any one of the preceding claims wherein the fragrance is present in an amount from 0.001 to 1.5% by weight of the personal care composition.

8. A composition according to any one of the preceding claims wherein the fragrance comprises an ingredient selected from the group consisting of alpha or beta pinene, myrcene, geranyl alcohol or acetate, camphene, dl-limonene, alpha or beta phellandrene, tricyclene, terpinolene, allocimmane, geraniol, nerol, linanool, dihydrolinanool, citral, ionone, methyl ionone, citronellol, citronellal, alpha terpineol, beta terpineol, alpha fenchol, borneol, isoborneol, camphor, terpinen-1-ol, terpin-4-ol, dihydroterpineol, methyl chavicol, anethole, 1,4 or 1,8 cineole, geranyl nitrile, isobomyl acetate, linalyl acetate, caryophyllene, alpha cedrene, guaiol, patchouli alcohol, alpha or beta santalol, ethylene brassylate and mixtures thereof.

9. A composition according to any one of claims 1 to 7 wherein the fragrance comprises an ingredient selected from the group consisting of 1-butanol, 3-methyl acetate, 2-buten-1-ol 3-methyl acetate, beta pinene, hexyl acetate, limonene, 2,6 dimethyl hept-5-en-1-al, gamma terpinene, dihydromyrcenol, 2,4 dimethyl 3 cyclohexene 1 carbaldehyde, linalool, benzyl acetate, allyl heptoate, 2-tertiobutylcydohexyl acetate-2, alpha isomethyl ionone and lily aldehyde.

10. A composition according to any one of claims 1 to 9 further comprising from 0.5 to 50% of glycerol by weight of the composition.

## Patentansprüche

1. Körperpflege-Zusammensetzung, umfassend:
(i) 0,0001 bis 5 % eines Duftstoffs, um einem menschlichen Körper, auf den die Zusammensetzung angewendet wird, einen angenehmen Geruch zu verleihen;
(ii) 0,01 bis 20 % einer substituierten Harnstoffverbindung der allgemeinen Formel (I) 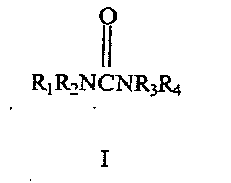 worin R₁, R₂ und R₃ aus der Gruppe, bestehend aus Wasserstoff, C₁-C₆-Alkyl, (R₅)ₙOH und Gemischen davon, ausgewählt sind; R₅ Methylen, Ethylen, Propylen oder Kombinationen davon ist und n im Bereich von 1 bis 6 liegt; und R₄ (R₅)ₙOH ist und
(iii) 0,01 bis 20 Gewichts-% eines quaternären Dihydroxypropyltrialkylammonium-Salzes der Struktur AB, worin A eine kationisch geladene Komponente des Salzes AB ist, B eine anionisch geladene Komponente des Salzes AB ist und A ein einzelnes quaternisiertes Stickstoffatom, wenigstens zwei Hydroxy-Gruppen und ein Molekulargewicht von nicht höher als 250 hat.

2. Zusammensetzung nach Anspruch 1, wobei das quaternäre Ammoniumsalz ein Dihydroxypropyltri(C₁-C₃-alkyl)-ammonium-Salz ist.

3. Zusammensetzung nach Anspruch 2, wobei das Salz Trihydroxypropyltrimoniumchlorid ist.

4. Zusammensetzung nach einem der vorangehenden Ansprüche, die aus der Gruppe, bestehend aus "Leave-on"-Hautlotionen und -cremes, Shampoos, Haarkonditionierern, Duschgelen, Toilettenseifen, Antiperspiranzien, Deodorants, dentalen Produkten, Rasiercremes, Enthaarungsmitteln, Lippenstiften, Grundierungen, Mascara, Selbstbräuner und Sonnenschutzlotionen, ausgewählt ist.

5. Zusammensetzung nach einem der vorangehenden Ansprüche, wobei die substituierte Harnstoffverbindung in einer Menge von 0,05 bis 15 Gewichts-% der Körperpflege-Zusammensetzung vorliegt.

6. Zusammensetzung nach einem der vorangehenden Ansprüche, wobei die substituierte Harnstoffverbindung Hydroxyethylharnstoff ist.

7. Zusammensetzung nach einem der vorangehenden Ansprüche, wobei der Duftstoff in einer Menge von 0,001 bis 1,5 Gewichts-% der Körperpflege-Zusammensetzung vorliegt.

8. Zusammensetzung nach einem der vorangehenden Ansprüche, wobei der Duftstoff ein Ingrediens, ausgewählt aus der Gruppe, bestehend aus alpha- oder beta-Pinen, Myrcen, Geranylalkohol oder -acetat, Camphen, dl-Limonen, alpha- oder beta-Phellandren, Tricyclen, Terpinolen, Allocimman, Geraniol, Nerol, Linanool, Dihydrolinanool, Citral, Ionon, Methylionon, Citronellol, Citronellal, alpha-Terpineol, beta-Terpineol, alpha-Fenchol, Borneol, Isoborneol, Campher, Terpinen-1-ol, Terpin-4-ol, Dihydroterpineol, Methylchavicol, Anethol, 1,4- oder 1-8-Cineol, Geranylnitril, Isobornylacetat, Linalylacetat, Caryophyllen, alpha-Cedren, Guaiol, Patchoulialkohol, alpha- oder beta-Santalol, Ethylenbrassylat und Gemischen davon, umfasst.

9. Zusammensetzung nach einem der Ansprüchen 1 bis 7, wobei der Duftstoff ein Ingrediens umfasst, das aus der Gruppe, bestehend aus 1-Butanol, 3-Methylacetat, 2-Buten-1-ol-3-methylacetat, beta-Pinen, Hexylacetat, Limonen, 2,6-Dimethyl-hept-5-en-1-al, gamma-Terpinen, Dihydromyrcenol, %,4-Dimethyl-3-cyclohexen-1-carbaldehyd, Linalool, Benzylacetat, Allylheptoat, 2-Tertiobutylcyclohexylacetat-2, alpha-Isomethylionon und Lilyaldehyd, ausgewählt ist.

10. Zusammensetzung nach einem der Ansprüche 1 bis 9, die außerdem 0,5 bis 50 % Glycerin, bezogen auf das Gewicht der Zusammensetzung, umfasst.

## Revendications

1. Composition de soins personnels comprenant:
(i) de 0,0001 à 5% d'un parfum pour donner une odeur agréable sur un corps humain sur lequel la composition est appliquée;
(ii) de 0,01 à 20% d'une urée substituée de structure générale (I) 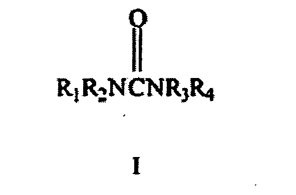 dans laquelle R₁, R₂ et R₃ sont choisis dans le groupe constitué par un atome d'hydrogène, un groupe alkyle en C₁ à C₆, (R₅) nOH et leurs mélanges ; R₅ est un groupe méthylène, éthylène, propylène ou des combinaisons de ceux-ci et n vaut de 1 à 6 ; et R₄ est (R₅)ₙOH ; et
(iii) de 0,01 % à 20% en poids d'un sel de dihydroxypropyltrialkyle ammonium quaternaire de structure AB, dans laquelle A est un composant à charge cationique du sel AB, B est un composant à charge anionique du sel AB, et A a un atome d'azote quaternisé unique, au moins deux groupes hydroxy et un poids moléculaire pas supérieur à 250.

2. Composition selon la revendication 1, dans laquelle le sel d'ammonium quaternaire est un sel de dihydroxypropyltri(alkyle en C₁ à C₃) ammonium.

3. Composition selon la revendication 2, dans laquelle le sel est le chlorure de dihydroxypropyltrimonium.

4. Composition selon l'une quelconque des revendications précédentes, qui est choisie dans le groupe constitué par les lotions et les crèmes à laisser sur la peau, les shampooings, les conditionneurs capillaires, les gels douche, les pains de toilette, les anti-transpirants, les déodorants, les produits dentaires, les crèmes à raser, les crèmes dépilatoires, les rouges à lèvres, les fonds de teint, les mascaras, les autobronzants et les lotions d'écran solaire.

5. Composition selon l'une quelconque des revendications précédentes, dans laquelle l'urée substituée est présente en une quantité de 0,05 à 15 % en poids de la composition de soins personnels.

6. Composition selon l'une quelconque des revendications précédentes, dans laquelle l'urée substituée est une hydroxyéthylurée.

7. Composition selon l'une quelconque des revendications précédentes, dans laquelle le parfum est présent en une quantité de 0,001 à 1,5 % en poids de la composition de soins personnels.

8. Composition selon l'une quelconque des revendications précédentes, dans laquelle le parfum comprend un ingrédient choisi dans le groupe constitué par les α - ou β -pinène, le myrcène, l'alcool ou l'acétate de géranyle, le camphène, le dilimonène, les α ou β-phellandrènes, le trïcyclène, le terpinolène, l'allocimane, le géraniol, le nérol, le linanool, le dihydrolinanool, le citral, l'ionone, la méthyl-ionone, le citronellol, le citronellal, l'α -terpinéol, le β-terpinéol, l'α-fenchol, le boméol, l'isoboméol, le camphre, le terpinèn-1-ol, le terpin-4-ol, le dihydroterpinéol, le méthylchavicol, l'anéthol, le 1,4 ou 1,8-cinéol, le géranylnitrile, l'acétate d'isobomyle, l'acétate de linalyle, le caryophyllène, l'α-cédrène, le guaïol, l'alcool de patchouli, les α - ou β - santalols, le brassylate d'éthylène et leurs mélanges.

9. Composition selon l'une quelconque des revendications 1 à 7; dans laquelle le parfum comprend un ingrédient choisi dans le groupe constitué par le 1-butanol, l'acétate de 3-méthyle, l'acétate de 2-butén-1-ol-3-méthyle, le β-pinène, l'acétate d'hexyle, le limonène, le 2,6-diméthyl-hept-5-én-1-al, le gamma-terpinène, le dihydromyrcénol, le 2,4-diméthyl-3-cyclohexène-1-carbaldéhyde, le linalool, l'acétate de benzyle, l'heptoate d'allyle, l'acétate-2 de 2-tertiobutylcyclohexyle, l'α-isométhylionone et le lily-aldéhyde.

10. Composition selon l'une quelconque des revendications 1 à 9, comprenant en outre de 0,5 à 50% de glycérol en poids de la composition.
